# EUROPEAN PATENT APPLICATION

(11) **EP 4 249 913 A1**
(43) Date of publication of application: **27.09.2023**
(21) Application number: 22305335.6
(22) Date of filing: 22.03.2022
(51) Int. Cl.: G01N 33/50, A61K 38/17, A61K 31/00, A61P 7/02, A61P 9/00, C07K 16/00

(54) **TOLL-LIKE-RECEPTOR 3 INHIBITOR FOR PREVENTING AND/OR TREATING DISORDERS IN PATIENTS WITH A PERSONAL HISTORY OF VENOUS THROMBOEMBOLISM (VTE)**

(71) Applicant: Centre Hospitalier Universitaire de Nîmes, 30900 Nîmes Cédex 9 (FR); Université de Montpellier, 34090 Montpellier (FR)
(72) Inventor: BOUVIER, Sylvie, 30000 NIMES (FR); GRIS, Jean-Christophe, 30000 NIMES (FR); FORTIER, Mathieu, 34670 BAILLARGUES (FR)
(74) Representative: Regimbeau

(57) **Abstract**

The present invention concerns a Toll-Like-Receptor 3 (TLR3) inhibitor for use in a method for preventing and/or treating disorders in patients with a personal history of venous thromboembolism (VTE), in particular a history of proximal VTE, and related pharmaceutical composition.

## Description

### TECHNICAL FIELD OF THE INVENTION

The present invention relates to the prevention and/or treatment of disorders in patients with a personal history of venous thromboembolism (VTE).

### BACKGROUND ART

Despite progress in antithrombotic prophylaxis, venous thromboembolic (VTE) disease remains an important public health issue, resulting in high morbidity and mortality. The risk of recurrence is significant and very few biomarkers have a real practical impact on the management of VTE disease or the evaluation of thrombosis-related cellular/tissue injury. Deregulation of immunological mechanisms plays an important role in the onset of venous thromboembolic complications such as deep vein thrombosis (DVT) and pulmonary embolism (PE). The antiphospholipid syndrome (APS) constitutes a model of autoimmune disease. It combines clinical manifestations (arterial or venous thrombosis, obstetric complications) with biological signs (Miyakis et al. 2006), the latest corresponding to the persistent presence of antibodies called "anti-phospholipids" (aPL): anti-cardiolipid (aCL), anti-β2 glycoprotein 1 (aβ2-GP1) and lupus anticoagulant (LAC).

Although the presence of aPL is a necessary pre-condition, APS-associated clotting is seemingly triggered by an additional 'second hit', frequently related to innate inflammatory immune responses. β2 glycoprotein I (β2GPI)-dependent aPL is described as the most important subset of these antibodies and mediate several thrombogenic mechanisms, mainly on the basis of their reactivity with β2GPI expressed on the membrane of cells that participate in the coagulation cascade (Meroni et al. Nat Rev Rheumatol 2011).

Among these cells, monocytes are a major player in vascular biology and in activating hemostasis. Under pathological circumstances, in response to inflammatory cytokines or platelet activation, they acquire a pro-coagulant phenotype, in particular by activating the transcription of the tissue factor gene. The expression of tissue factor (TF) on monocytes surface then activates the coagulation system contributing to the occurrence of thrombosis (Rao & Pendurthi 2012). Several mechanisms have been proposed to explain the pathophysiology of APS: activation of the complement system, involvement of cellular receptors and ultimately activation of intracellular signaling pathways (Vega-Ostertag et al. 2005; López- Pedrera et al. 2006; Canaud et al. 2014). Preliminary studies, carried out *in vitro* from monocyte cultures and comparing APS patients with vascular manifestations to healthy controls, revealed differences in the activation of signaling pathways involving protein kinases (MAPKs), the NFκB nuclear factor transcription, the MEK-1/ERK pathway and in the expression of genes involved in coagulation (Lopez-Pedrera et al. 2006, 2010; Cuadrado et al. 2006).

In the context of personalized medicine, the search for biological risk factors for VTE is based on carrying out a "thrombophilia workup", looking for constitutional (genes-related) or acquired (APS essentially) abnormalities of coagulation. However, in daily clinical practice, it may happen to find patients with a clinical profile who have a biological assessment persistently "hopelessly" negative. A reason may be due to the limitations of traditional technical approaches or to the existence of antigenic targets other than those known.

So there is still a need to understand biological abnormalities and mechanism associated with VTE and consequently to provide targeted molecular therapies based on intracellular molecular mechanisms for treating patients with a personal history of proximal VTE, in particular for aPL negative patients with a history of proximal VTE.

The inventors demonstrated that certain immunoglobulins G (IgG), present in the plasma of aPL negative patients with a history of proximal VTE, interact with Toll-Like-Receptor 3 (TLR3 receptor) bound to the monocyte membrane and activate ERK signaling pathways that ultimately promote the expression of pro-inflammatory and pro-coagulant factors. These results open a new way to prevent and/or treat disorders in patients with a personal history of VTE, using TLR3 inhibitor.

### SUMMARY OF THE INVENTION

A first subject-matter of the present invention is a Toll-Like-Receptor 3 (TLR3) inhibitor for use in a method for preventing and/or treating disorders in patients with a personal history of venous thromboembolism (VTE), in particular a history of proximal VTE.

The present invention also relates to a pharmaceutical product or composition comprising a TLR3 inhibitor and one or more active compounds selected from the group consisting of:
(i) intracellular partner inhibitor inducing the activation of the ERK signaling pathway,
(ii) anti-thrombotic agent, and
(iii) immunomodulatory agent.

Another subject-matter of the present invention is the pharmaceutical product according to the invention for use in a patient in need thereof for preventing and/or treating disorders linked to venous thromboembolism (VTE).

The present invention also concerns an *in vitro* method for identifying aPL negative patients with a personal history of VTE that may benefit of the treatment with a TLR3 inhibitor as defined in the invention or of the treatment with a pharmaceutical product as defined in the invention, comprising the steps of :
a) Selecting patients with a personal history of VTE, preferably with proximal VTE,
b) Screening the antiphospholipid antibodies negative patients (aPL-) from individuals of step a),
c) Purifying IgG from a plasma sample of the aPL negative patient of step b),
d) Contacting the IgG of step c) with a cultured monocyte line, as THP-1 line,
e) Measuring the expression of phospho-ERK1/2, ERK1/2 and actin as control, and
f) Identifying the patient that may benefit of the treatment with a TLR3 inhibitor according to the increase expression of phospho-ERK1/2 in comparison to the expression of ERK1/2 and actin.

Another subject-matter of the present invention is a kit for carrying out the *in vitro* method as defined above for identifying aPL negative patients with a personal history of VTE comprising :
- A culture monocyte line, in particular THP-1 line,
- Primary and secondary antibodies targeting phospho-ERK1/2, ERK1/2 or actin, or alternatively a reporter gene with fluorescent or luminescent protein able to reveal phospho-ERK1/2 activity
- Reagents for cell lysis, phosphatase and protease inhibitors,
- Gel, membrane, buffers, and molecular weight ladder.

### DESCRIPTION OF THE FIGURES

**Figure 1****: Participant Flowchart**
**Figure 2****: ERK pathway is activated by IgG isolated from aPL negative patients**
   A) Representative Western Blot of ERK activation in aPL negative patients. Increase of Phospho-ERK was not due to an increased expression of total ERK.
   B) Western blot quantification. Ratio of Phospho ERK to Actin were normalized to the corresponding controls for each experiment.
**Figure 3****: ERK pathway activation in aPL negative patients is mediated by TLR3**
   A) Representative Western Blot of ERK activation, in presence or not of TLR3 blocking antibody.
   B) Western blot quantification. Ratio of Phospho ERK to Actin were normalized to the corresponding controls for each experiment. Increase of Phospho-ERK signal was completely abolished in presence of TLR3 blocking antibody.

### DETAILED DESCRIPTION OF THE INVENTION

Thrombosis that occurs spontaneously or secondary to a risk factor usually starts in the deep veins of the calfs. Some of risk factors are synthetic estrogen intake, pregnancy, older age, malignancy, surgery, inflammatory disorders and inherited thrombophilia. Most deep vein thrombosis (DVT) resolves spontaneously, but some extends to the proximal veins and subsequently break free to cause pulmonary embolism (PE).

Each of these stages of venous thromboembolism (VTE), e.g. calf DVT, proximal DVT, PE, may or may not be associated with symptoms. The development of symptoms depends on the extent of thrombosis, the adequacy of collateral vessels, and the severity of associated vascular occlusion and inflammation. DVT is usually detected by ultrasound techniques applied to the vascular venous tree of the leg (Echo/Doppler), Concerning PE, computed tomography, or CT scan, or CAT scan are most often used.

The risk of progression of VTE is greater when there are continuing risk factors for thrombosis (such as immobilization after surgery, presence of inherited thrombophilia) and when the initial thrombosis is large.

Antithrombotic prophylaxis facilitates spontaneous lysis of perioperative DVT and prevents thrombi form forming. But the risk of recurrence remains elevated after a first episode of VTE and particularly after a proximal unprovoked event.

So there is still a need for new therapies in the prevention and/or treatment of patients with a personal history of VTE, in particular with a history of proximal VTE whereas they have a biological assessment persistently negative (especially for aPL negative patients). And the inventors presently demonstrated the role of TLR3 in the ERK signaling pathway that promotes the expression of pro-inflammatory and pro-coagulant factors.

### TLR3 inhibitor and uses in patients with a personal history of VTE

The present invention concerns a Toll-Like-Receptor 3 inhibitor for use in a method for preventing and/or treating disorders in patients with a personal history of venous thromboembolism (VTE), in particular a history of proximal VTE.

TLRs are type I transmembrane proteins composed of an extracellular domain of leucinerich repeats and an intracellular Toll/interleukin-1 (IL-1) receptor (TIR) domain. Mammalian TLRs comprise a large family consisting of at least 10 functional members such as TLR1-9 which are conserved between the human and mouse. TLRs play a central role in host innate and acquired immunity, as well as in pathogenesis of various diseases, including infectious diseases, inflammatory diseases and autoimmune diseases. TLR3 is in particular implicated in the recognition of dsRNA and viruses and the antiviral gene response thereto.

By **'TLR3 inhibitor',** it means an inhibitor that acts as a direct, competitive and high affinity inhibitor of ligand binding to TLR3.

By 'high affinity inhibitor of ligand binding to TLR3', it means an inhibitor with an inhibitory constant in the micromolar range or lower.

A TLR3 inhibitor that is convenient for use in the present invention may be selected according to an *in vitro* method comprising the steps of :
1) Culturing monocyte cells line, in particular THP-1 cells,
2) Adding the potential inhibitor to be tested for a dose-response experiment,
3) Adding IgG, in particular for 15 minutes,
4) Preparing samples for Western Blot and analyzing the P-Erk/ actin ratio values according to the different concentrations of the inhibitor used in step 2,
5) Identifying and selecting TLR3 inhibitor having a decrease of P-Erk/ actin ratio.

As used herein, the terms **"treating" or "treatment"** refers to therapeutic treatment, wherein the object is to prevent or slow down (lessen) the targeted disease or disorder in patient with a personal history of VTE. A patient is successfully "treated" for a particular disease or disorder, if after receiving a therapeutic amount of the TLR3 inhibitor according to the invention, the patient shows observable and/or measurable reduction in or absence of one or more signs and symptoms of said disease or disorder.

As used herein, the terms **"prevent", "prevention" and "preventing"** refer to the reduction in the risk of acquiring or developing a given condition, or the reduction or inhibition of the recurrence or said condition in an individual who is not presently ill, but who has been (has a personal history of VTE) or may be near a patient with the disease.

In particular, the TLR3 inhibitor is used for preventing the induction of, or inhibiting the progression of a venous thrombotic disorder.

It is also to be appreciated that the various modes of treatment or prevention of medical conditions as described are intended to mean "substantial," which includes total but also less than total treatment or prevention, and wherein some biologically or medically relevant result is achieved.

By **'patients with a personal history of venous thromboembolism (VTE)',** it means patients that already had a first episode of VTE and considered as having a risk of recurrence. The risk of recurrence is higher in patients who developed a proximal unprovoked event.

By '**patients with a personal history of proximal VTE',** it means patients that already had a first episode of VTE involving proximal deep vein thromboses (DVT), in particular in legs from the popliteal vein, and/or pulmonary embolism.

The patients may have additional risk factors for thrombosis or not. Indicative (but not exhaustive) such cases include age (>55 in men and >65 in women), and the presence of the established risk factors for cardiovascular disease (hypertension, diabetes mellitus, elevated LDL or low HDL cholesterol, cigarette smoking, family history of premature cardiovascular disease, body mass index superior or equal to 30kg m-2, microalbuminuria), inherited thrombophilias, oral contraceptives with estrogens, pregnancy, nephrotic syndrome, malignancy, immobilization and surgery.

By **'disorders'** in patients with a personal history of VTE, it means cardiovascular disorders and/or complications that may extend to pulmonary embolism and death.

In a particular embodiment, the patient is an antiphospholipid antibodies (aPL) negative patient.

By '**aPL-patient' or 'aPL negative patient',** it means a **patient** that is negative for conventional antiphospholipid antibodies (aPL) among IgM/IgG Anti-cardiolipid (aCL), IgM/IgG anti-β2 glycoprotein-l (aβ2GPI) antibodies, and Lupus anticoagulant (LAC). These aPL are usually quantified according to the Sapporo criteria updated in Sydney in 2006 which are those currently recommended to diagnose Anti-Phospholipid Syndrome APS (Miyakis et al. JTH 2006). In a particular embodiment, LAC may be screened by coagulation methods using 2 reagents (PTT-LA^{®}: an aPTT reagent sensitized to the detection of LA; and Staclot-DRVVT^{®}: a dilute Russel viper venom time DRVVT reagent, Stago, Asnières, France) and aCL and aβ2GPI, which are solid-phase antibodies, may be quantified by ELISA according to the manufacturer's instructions (For example Orgentec, Trappes, France).

By 'negative patient', it means that the levels of IgM/IgG Anti-cardiolipid (aCL), IgM/IgG anti-β2 glycoprotein-l (aβ2GPI) antibodies, and Lupus anticoagulant (LAC) are lower than the ones detected for positive patients according to laboratory criteria as disclosed in Miyakis et al, 2006:
- Lupus anticoagulant (LA) present in plasma, on two or more occasions at least 12 weeks apart, detected according to the International Society on Thrombosis and Haemostasis ;
- Anticardiolipin (aCL) antibody of IgG and/or IgM isotype in serum or plasma, present in medium or high titer (ie > 40 GPL or MPL, or > 99^{th} percentile), on two or more occasions, at least 12 weeks apart, measured by a standardized ELISA;
- Anti-β2 glycoprotein-l antibody of IgG and/or IgM isotype in serum or plasma (in titer > the 99^{th} percentile), preset on two or more occasions, at least 12 weeks apart, measured by a standardized ELISA, according to recommended procedures.

As used herein, the terms 'TLR3 inhibitor' encompasses any compound inhibiting the ligand binding to TLR3.

In particular, the TLR3 inhibitor is selected from the group consisting of a protein, a polypeptide, a peptide, a lipid, a natural or synthetic polymer, a polysaccharide, and any compound inhibiting the ligand binding to TLR3.

As non-limitative examples of protein, polypeptide or peptide, mention may be made of protamine, an antibody, a chimeric peptide. Specific peptides and antibodies are disclosed hereunder.

As non-limitative examples of lipid, mention may be made of poly(L-lysine) or poly(amidoamine) (PAMAM) dendrimer generation 4.

As non-limitative examples of polymer, mention may be made of β-cyclodextrin-containing polycation (CDP), polyphosphoramide polymer (PPA-DPA), poly(amidoamine) (PAMAM) dendrimer generation 4, or hexadimethrine bromide (HDMBr also known as polybrene). As non-limitative examples of polysaccharide, mention may be made of *Astragalus* polysaccharide or *Radix isatidis* polysaccharide.

As illustrative but non-limitative examples, the inhibitor is selected from the group consisting of: a TLR3 blocking peptide such as DAG-P1969, a monoclonal antibody designed on the extra-cellular domain of TLR3 such as an anti-TLR3 ADCC Enhanced Antibody IPH33-1, or the antibody TLR3.7, and a molecule such as R)-2-(3-Chloro-6-fluorobenzo[b]thiophene-2-carboxamido)-3-phenylpropanoic acid or *N*-[(3-Chloro-6-fluorobenzo[*b*]thien-2-yl)carbonyl]-D-phenylalanine.

In a particular embodiment, the TLR3 inhibitor is a TLR3 blocking peptide (DAG-P1969) from Creative Diagnostics.

In another particular embodiment, the TLR3 inhibitor is an Anti-TLR3 ADCC Enhanced Antibody (IPH33) commercialized by Innate Pharma, that is an ADCC enhanced antibody produced by our Afuco^{™} platform. IPH33 is a monoclonal antibody targeting the Toll-like Receptor-3 receptor (TLR3); designed on the extra cellular domain of TLR3.

In another particular embodiment, the TLR3 inhibitor is a TLR3 Antibody (N-term) Blocking Peptide from Clinisciences.

In another particular embodiment, the TLR3 inhibitor is a TLR3/dsRNA Complex Inhibitor from Calbiochem : TLR3/dsRNA Complex Inhibitor acts as a direct, competitive and high affinity inhibitor of dsRNA binding to TLR3 (Ki = 2.96 µM; Kd = 19 nM). Selectively antagonizes stimulated TLR3 signaling. (R)-2-(3-Chloro-6-fluorobenzo[b]thiophene-2-carboxamido)-3-phenylpropanoic acid.

In another particular embodiment, the TLR3 inhibitor is a CU CPT 4a from Bio-Techne, that is a selective TLR3 inhibitor (IC50 = 3.44 µM in RAW 264.7 cells); suppresses downstream signaling pathways mediated by the TLR3/dsRNA complex, inhibiting TNF-α and IL-1β production in whole cells. Reduces death of crypt cells and improves gastrointestinal syndrome in mice. *N*-[(3-Chloro-6-fluorobenzo[*b*]thien-2-yl)carbonyl]-D-phenylalanine.

In another particular embodiment, the TLR3 inhibitor is an anti-TLR3 antibody [TLR3.7] (ab12085) *Abcam* Recombinant full length protein (DDDDK-tag) corresponding to Human TLR3. Human DDDDK-tagged TLR3 stably expressed by Ba/F3 cells. The biological activity of the antibody has been described in Matsumoto, M et al; 2002. Monoclonal antibody TLR3.7 inhibits dsRNA-induces IFN-beta production.

Any other commercialized TLR3 inhibitor or TLR3 inhibitor identified by a screening test as disclosed above may be used in the present invention.

In particular, the inhibitor suppresses downstream signaling pathways mediated by the TLR3/ligand complex, inhibiting the ERK pathways of thrombosis and haemostasis-competent cells such as monocytes activation and production of pro-inflammatory and procoagulants agents.

### Pharmaceutical product and uses

The TLR3 inhibitor may be combined with pharmaceutically acceptable excipients.

**"Pharmaceutically" or "pharmaceutically acceptable"** refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical products or compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. In particular, sterile aqueous solutions are especially suitable for intravenous, intramuscular, subcutaneous and intraperitoneal administration.

The TLR3 inhibitor may be utilized in combination with one or more other drugs in the treatment, prevention, control, amelioration, or reduction of risk of diseases or conditions related to VTE, where the combination of the drugs together are safer or more effective than either drug alone.

So another subject-matter of the present invention is a pharmaceutical product (also named a 'combination product') or a composition comprising a TLR3 inhibitor and one or more active compounds selected from the group consisting of:
(i) intracellular partner inhibitor inducing the activation of the ERK signaling pathway,
(ii) anti-thrombotic agent, and
(iii) immunomodulatory agent.

### Intracellular partner inhibitor

In a particular embodiment, the active compound combined to TLR3 inhibitor is an intracellular partner inhibitor inducing the activation of the ERK signaling pathway, in particular Pepinh-TRIF (Invivogen) that is a 30 aa peptide that blocks TRIF signaling by interfering with TLR-TRIF interaction.

### Anti-thrombotic agent

In another particular embodiment, the active compound combined to TLR3 inhibitor is an anti-thrombotic agent commonly used in the treatment of venous thromboembolism (VTE), such as the ones disclosed in the reference Sanchez O et al., 2019. Mention may be made of low molecular heparin (LMWH), vitamin K antagonist (VKA), direct oral anti-coagulant (DOAC), unfractionated heparin, fondaparinux and mixtures thereof.

### Immunomodulatory agent.

In another particular embodiment, the active compound combined to TLR3 inhibitor is an immunomodulatory agent, in particular an immunomodulatory agent reducing the production of IgG by lymphocytes B and/or reducing the stimulation of ERK-signaling pathway by IgG. Mention may be made of rituximab, belimumab, polyvalent gamma-globulins, humanized immunoglobulins fragments inhibiting the binding of IgG, and mixtures thereof.

The skilled in the art will combine the TLR3 inhbitor and one or more active compounds as cited above with adapted doses and routine of administration depending the condition of the patient to be treated.

In a particular embodiment, as illustrative but non-limitative examples, the TLR 3 inhibitor is selected from the group consisting of a peptide or an antibody targeting TLR3 and the active compound is selected from the group consisting of a peptide blocking TRIF signaling by interfering with TLR-TRIF interaction, low molecular heparin (LMWH), vitamin K antagonist (VKA), direct oral anti-coagulant (DOAC), unfractionated heparin, fondaparinux, rituximab, belimumab, polyvalent gamma-globulins, and humanized immunoglobulins fragments inhibiting the binding of IgG.

The TLR3 inhibitor and the one or more active compounds may be provided for simultaneous, sequential or delayed administration.

The pharmaceutical product (combination product) or the composition comprises a pharmaceutical excipient, as disclosed above.

According to the invention, the TLR3 inhibitor is administered to the patient in need thereof with a therapeutically effective amount.

By a **"therapeutically effective amount"** is meant a sufficient amount of the TLR3 inhibitor to prevent a risk of recurrence of VTE or treat the disease or disorder (e.g. VTE) at a reasonable benefit/risk ratio applicable to any medical treatment. The specific therapeutically effective dose level for any particular patient in need thereof will depend upon a variety of factors including the age, body weight, general health, sex and diet of the patient in need thereof; the time of administration, route of administration, and rate of excretion of the TLR3 inhibitor and optional additional active compounds employed; the duration of the treatment; drugs used in combination or coincidental with the specific TLR3 inhibitor employed; and like factors well known in the medical arts.

The present invention also concerns the pharmaceutical product according to the invention for use in a patient in need thereof for preventing and/or treating disorders linked to venous thromboembolism (VTE).

The patient in need thereof according to the invention is a patient with a personal history of VTE, in particular a history of proximal VTE, as disclosed above. The patient may be symptomatic or asymptomatic, with a risk of recurrence to spontaneous VTE or a risk of VTE induced by an external factor as described previously.

As disclosed above, the patient in need thereof may also have additional risk factors for thrombosis, such as age (>55 in men and >65 in women), and the presence of the established risk factors for cardiovascular disease (hypertension, diabetes mellitus, elevated LDL or low HDL cholesterol, cigarette smoking, family history of premature cardiovascular disease, body mass index superior or equal to 30kg m-2, microalbuminuria), inherited thrombophilias, oral contraceptives, nephrotic syndrome, malignancy, immobilization and surgery.

In a particular embodiment, the patient in need of the treatment of the invention is an aPL negative patient.

In a particular embodiment, the aPL negative patient in need thereof has been identified by an *in vitro* method comprising the steps of :
a) Selecting patients with a personal history of VTE, preferably with proximal VTE,
b) Screening the antiphospholipid antibodies (aPL) negative patients from individuals of step a),
c) Purifying IgG from a plasma sample of the aPL negative patient of step b),
d) Contacting the IgG of step c) with a cultured monocyte line, as THP-1 line,
e) Measuring the expression of phospho-ERK1/2, native protein ERK1/2 and actin as control, and
f) Identifying the patient that may benefit of the treatment with a TLR3 inhibitor according to the increase expression of phospho-ERK1/2 in comparison to the expression of native protein ERK1/2 and actin.

The step a) comprises a selection of patient with a personal history of VTE, preferably with proximal VTE. This selection is made on medical background of the patient.

The step b) comprises a screening of patients that are negative for conventional antiphospholipid antibodies (aPL) among IgM/IgG anti-cardiolipid (aCL), IgM/IgG anti-β2 glycoprotein-l (aβ2GPI) antibodies, and Lupus anticoagulant (LAC), according to the Sapporo criteria updated in Sydney in 2006 which are those currently recommended to diagnose Anti-Phospholipid Syndrome APS (Miyakis et al. JTH 2006). In a particular embodiment, LAC may be screened by 2 coagulation reagents (PTT-LA^{®}: an aPTT reagent sensitized to the detection of LA; and Staclot-DRVVT^{®}: a dilute Russel viper venom time DRVVT reagent, Stago, Asnières, France) and aCL and aβ2GPI may be quantified by ELISA or any other convenient solid-phase technqiue according to the manufacturer's instructions (for example Orgentec, Trappes, France).

The step c) comprises purifying IgG from a plasma sample, according to well-known methods of protein purification. In a particular embodiment, IgG are purified using protein G agarose beads.

The step d) comprises contacting the IgG of step c) with a cultured monocyte line, as THP-1 line, according to well-known methods.

In particular, cells were seeded in a well plate, preferably 1.10⁶ cells/well were seeded in a 6-well plate. The day after, cells were pre-stimulated with LPS, in particular 5mg/mL for 30 minutes. After centrifugation, media was replaced in particular by FBS free RPMI and cells were stimulated with control or patient IgG, in particular at 100µg/mL for 15 minutes.

The step e) comprises measuring the expression of phospho-ERK1/2, native protein ERK1/2 and actin as control, according to well-known methods.

In particular embodiment, the expression of proteins is measured by a Western blot analysis and use of primary and secondary antibodies targeting phospho-ERK1/2, ERK1/2 or actin. In particular, the protein extracts are loaded on a polyacrylamide gel, and transferred on a membrane. The membrane is further incubated with primary P-ERK or total ERK antibodies and, after washing, use of an appropriate secondary antibody. Signals were finally revealed using a solution adapted to reveal the primary-secondary antibodies complex. A control is made with anti-actin antibody and the appropriate anti secondary antibody. The secondary antibody may be labelled or coupled to an enzyme (eg HRP) that may be revealed by a solution containing its substrate.

Quantifications are made with an adapted software.

In another particular embodiment, the expression of proteins is measured by a reporter gene with fluorescent or luminescent protein revealing an increase of phospho-ERK1/2 activity.

Commonly used reporter genes that induce visually identifiable characteristics usually involve fluorescent and luminescent proteins. Mention may be made of Green fluorescent protein (GFP) that causes cells that express it to glow green under UV light; and Luciferase enzyme that catalyzes a reaction with its substrate (usually luciferin) to produce yellow-green or blue light, depending on the luciferase gene.

GFP-based, such as the ones described in Maryu et al. (Cell Struct Funct, 2018), or luciferase-based reporter genes will be transiently or constitutively expressed in THP-1. These cells will then be stimulated with IgG, as described before, and GFP or luciferase expression change will be quantified relatively to an appropriate control. Patients who have IgG inducing the highest changes would be the best candidates for TLR3 treatment.

The results are measured by a fluorescent microscope for GFP and a luminometer for luciferase.

The step f) comprises identifying the patient that may benefit of the treatment with a TLR3 inhibitor according to the increase expression of phospho-ERK1/2 in comparison to the expression of native protein ERK1/2 and actin.

In particular, we compare the normalized ratio Phospho-ERK/Actin with the ratio ERK/Actin and select the patients having an increase of the ratio Phospho-ERK/Actin corroborating an increase in the activation of ERK signaling pathway in patients with a personal history of proximal VTE and that may benefit of the treatment with a TLR3 inhibitor according to the invention.

The present invention also concerns a method of preventing the induction of, or inhibiting the progression of a thrombotic disorder in a patient with a personal history of VTE, in particular an aPL negative patient with history of VTE in need thereof, comprising administering to the patient a therapeutically effective amount of a TLR3 inhibitor and optionally one or more active compounds as disclosed above.

The present invention also concerns a method of treating a patient with a personal history of VTE, in particular an aPL negative patient with history of VTE in need thereof, comprising administering to the patient a therapeutically effective amount of a TLR3 inhibitor and optionally one or more active compounds as disclosed above.

In particular, the administration may comprise intravenous, intramuscular, subcutaneous, intraperitoneal or *per os* administration.

### In vitro method for identifying aPL negative patient with a personal history of VTE and kits

Another subject-matter of the present invention relates to an *in vitro* method for identifying aPL negative patients with a personal history of VTE that may benefit of the treatment with a TLR3 inhibitor as defined above or of the treatment with a pharmaceutical product (combination product) as defined above, comprising the steps of :
a) Selecting patients with a personal history of VTE, preferably with proximal VTE,
b) Screening the antiphospholipid antibodies (aPL) negative patients from individuals of step a),
c) Purifying IgG from a plasma sample of the aPL negative patient of step b),
d) Contacting the IgG of step c) with a cultured monocyte line, as THP-1 line,
e) Measuring the expression of phospho-ERK1/2, ERK1/2 and actine as control, and
f) Identifying the patient that may benefit of the treatment with a TLR3 inhibitor according to the increase expression of phospho-ERK1/2 in comparison to the expression of ERK1/2 and actin.

In a particular embodiment, the step e) uses Western Blot and primary and secondary antibodies targeting phospho-ERK1/2, ERK1/2 or actin.

In another particular embodiment, the step e) uses a reporter gene with fluorescent or luminescent protein revealing an increase of phospho-ERK1/2 activity.

The details for each step and related methods are disclosed above.

The present invention also relates to a kit for carrying out the *in vitro* method as defined above for identifying aPL negative patients with a personal history of VTE comprising :
- A culture monocyte line, in particular THP-1 line,
- Primary and secondary antibodies targeting phospho-ERK1/2, ERK1/2 or actine,
- Reagents for cell lysis, phosphatase and protease inhibitors,
- Gel, membrane, buffers, and molecular weight ladder.

As illustrative and non-limitative example, a kit for carrying out the *in vitro* method as defined above for identifying aPL negative patients with a personal history of VTE comprises:
- A THP-1 line (TIB-202, ATCC)
- Primary P-ERK rabbit antibody, primary total ERK rabbit antibody, goat-anti rabbit HRP secondary antibody; mouse anti-actin antibody and goat-anti mouse HRP secondary antibody,
- Reagents for cell lysis, phosphatase and protease inhibitors,
- Gel, membrane, buffers, and molecular weight ladder.

Alternatively, the present invention also relates to a kit for carrying out the *in vitro* method as defined above for identifying aPL negative patients with a personal history of VTE comprising :
- A culture monocyte line, in particular THP-1 line, and
- Reporter gene with fluorescent or luminescent protein able to reveal phospho-ERK1/2 activity.

As illustrative and non-limitative example, a kit for carrying out the *in vitro* method as defined above for identifying aPL negative patients with a personal history of VTE comprises:
- A THP-1 line (TIB-202, ATCC)
- Reporter gene with fluorescent protein able to reveal phospho-ERK1/2 activity, such as FIRE (Fra-1 based integrative reporter of ERK) degradation based system or KTR (kinase translocation reporter) , and
- Reporter gene with luminescent protein able to reveal phospho-ERK1/2 activity, such as the commercial kit MAPK/ERK Pathway SRE Reporter Construct, Firefly Luciferase from Promega.

The present invention will be now illustrated with the non-limitative examples.

### EXAMPLES

### 1. MATERIALS AND METHODS

### Participants

We performed a pilot prospective diagnostic study (Phase I) using a case-control design (Sackett et al. BMJ 2002) at the Hematology Outpatient Department at Nîmes University Hospital (France) ("ACTIMON" study, clinicaltrials.gov identifier: NCT02713581). As there is no data concerning monocytes activation by total IgG, we performed a preliminary study on a low number of patients with a personal history of VTE events recruited in our hematology outpatient department between March and May 2017.

We included 20 healthy women (Group Control, C) and 13 women who had a history of proximal venous thrombosis (Group Patient, P): proximal deep vein thrombosis (DVT) and/or pulmonary embolism (PE). The 20 healthy participants were recruited after a general call to all hospital staff. Ages of controls were matched with the ages of the patients. Exclusion criteria were pregnancy and postpartum period (up to 3 months after childbirth), chronic disease, chronic or recent infection and cancer for all groups, and specifically any history of thrombotic events for the control group.

The study was approved by the local Comité de Protection des Personnes soumises a la Recherche Biomédicale (CPP Sud Méditerranée III). This clinical investigation was performed in accordance with the 1996 revised version of the 1975 Helsinki Declaration. All participants had given written informed consent to participate in the trial and to constitute a biobank to be used later for the same theme. We collected the patients' clinical data: age, body mass index (BMI), smoking habits, history of obstetrics events, history of thrombotic events (anatomical and circumstantial classification), history of VTE in first-degree relatives and the use of antiplatelet or anticoagulant treatments or hormonal contraception.

### Participants' samples

After systematically discarding the first 5 mL of blood after puncture, citrated anticoagulated plasma was collected by a clean venipuncture procedure avoiding tourniquet. A double centrifugation procedure with 2.500 g for 15 minutes at 15°C was performed less than 1 hour after sampling. Aliquots were stored under sterile conditions at -80°C until coagulation tests and IgG purification.

EDTA anticoagulated blood was collected for blood cell count and the search for genetic risk factors for thrombosis.

Non-anticoagulated serum was collected for the quantification of non-conventional antiphospholipid antibodies and biochemical assays.

### Assays

### Standard coagulation assays

These assays were performed according to the publication of Gris et al. (2022). Standard coagulation tests (prothrombin time (PT, STA-NeoPTimal^{®}, Stago), activated partial thromboplastin time (aPTT^{®}, Stago), Clauss fibrinogen activity (Fg, STA-LiquidFib^{®}), study of intrinsic pathway (factors VIII, IX, XI and XII, STA-ImmunoDef^{®}, Stago), antithrombin (AT, StaChrom ATIII^{®}), proteins S and C assay (respectively STA-Staclot Protein S^{®} and STA-Staclot Protein C^{®}, Stago) were performed on the STA R Max^{®} automated coagulometer (Stago, Asnières, France) using the dedicated reagents, including calibrators, control plasmas and a reference normal plasma (normal human plasma pool: Pool Norm), according to the manufacturer's instructions. All these assays used a chronometric method except the quantification of antithrombin activity (chromogenic method).

### Antiphospholipid antibodies (aPLs)

We quantified conventional antiphospholipid antibodies according to the Sapporo criteria updated in Sydney in 2006 which are those currently recommended to diagnose AntiPhospholipid Syndrome APS (Miyakis et al. JTH 2006). They include high values of at least one aPL among IgM/IgG Anti-cardiolipids (aCL), IgM/IgG anti-β2 glycoprotein-I (aβ2GPI) antibodies, and Lupus anticoagulant (LAC). LAC was screened by 2 reagents (PTT-LA^{®}: an aPTT reagent sensitized to the detection of LAC; and Staclot-DRVVT^{®}: a dilute Russel viper venom time DRVVT reagent, Stago, Asnières, France). aCL and aβ2GPI were quantified by ELISA according to the manufacturer's instructions (Orgentec, Trappes, France).

As our patients were negative for conventional antiphospholipid antibodies, we also investigated nonconventional antiphospholipid IgG as described previously (Bardin N et al. Immunobiology 2007; Sanmarco et al. Thromb Haemost 2001).

### Genetic assays

Prothrombin G20210A (rs1099963) and factor V Leiden (rs6025) gene mutations were performed by allele-specific polymerase chain reaction (PCR) according to the manufacturer's instructions (respectively PTH 20210G>A RealFast^{™} Assay and and FV Leiden RealFast^{™} Assay, AmpliTech, Compiègne, France) on the LightCycler 480 (Roche, Meylan, France).

### Blood cell count

Complete blood and leukocyte counts were performed on the Sysmex XN-3000^{®} analyzer (Sysmex France, Villepinte).

### Other biological markers

C-reactive protein (CRP) quantification was carried out by Tina-quant^{®} C-reactive protein Gen.3 assay (Cobas^{®}, Roche Diagnostics, Meylan, France) according to the manufacturer's recommendations. Activities of L-alanine aminotransferase (ALAT), L-aspartate aminotransferase (ASAT), gamma-glutamyltransferase (GT) and creatinine assay were determined according to the recommendations of the manufacturer on the Cobas^{®} (Roche Diagnostics, Meylan, France).

### IgG purification

IgG were purified using protein G agarose beads according to manufacturer's protocol (reference 20397, Thermo scientific, Montigny-le-Bretonneux, France). Eluted IgG were then dialyzed overnight using 3 mL Thermo Scientific^{™} Slide-A-Lyzer^{™} G2 Dialysis Cassette with a 10K cut-off. Purified IgG were finally quantified using the Optilite system from Binding Site (Saint-Egrève, France).

### Cell culture and sample preparation

THP-1 (TIB-202, ATCC) were cultured in RPMI 1640 (Dutscher, Brumath, France) supplemented with 10% heat-inactivated fetal bovine serum (FBS) (Eurobio, Courtaboeuf, France). Cell cultures were maintained under standard culturing conditions (37°C, 5% CO₂, humidified atmosphere).

For Western blot sample, 1.10⁶ cells/well were seeded in a 6-well plate. The day after, cells were prestimulated with 5mg/mL LPS for 30 minutes. After centrifugation, media was replaced by FBSfree RPMI and cells were stimulated with control or patient IgG at 100µg/mL for 15 minutes. Cells were pelleted, washed with PBS and then lysed with RIPA buffer for 30 minutes on ice (Thermo scientific). Lysates were centrifuged for 30 minutes at 4°C and supernatants were then denatured for 5 minutes at 95°C, after addition of 5X denaturant loading buffer (Dutscher, Brumath, France). The exact same procedure was used for blocking TLR3 experiment, except that the mouse monoclonal [TLR3.7] (Abcam, Cambridge, United Kingdom) was added to the cells 30 minutes before the stimulation with IgG.

### Western Blot analysis

40µl of protein extracts were loaded on 4-12% polyacrylamide gel (Dutscher, Brumath, France) and transferred for 45 minutes on PVDF membrane (GE HealthCare) using the BlueBlot SD17 system (SERVA, Heidelberg, Germany). Membranes were blocked with Tris buffered saline with Tween 20 (TBST) with 1% BSA and then incubated with P-ERK or total ERK rabbit antibodies (Ozyme, Saint-Cyr-L'École, France) at 4°C overnight. The day after, membranes were washed with TBST and incubated with goat-anti rabbit HRP secondary antibody for 1h at room temperature RT. Signals were finally revealed using ECL prime solution (GE HealthCare, Buc, France) and mini HD9 system (Uvitec, Cambridge, UK). Bound antibodies were removed using Restore stripping solution (Thermo scientific, Montigny-le-Bretonneux) and after washes and saturation with 5% milk TBST, membranes were incubated with mouse anti actin antibody (Proteogenix, Schiltigheim, France) at 4°C overnight. The same procedure as previously was used with the appropriate goat-anti mouse HRP secondary antibody (Proteogenix, Schiltigheim, France). Quantifications were done using FIJI software.

### Statistics

The statistical analysis was performed with R 3.5.1 software (R Development Core Team [2018], R Foundation for Statistical Computing, Vienna, Austria) and GraphPad statistical software program (version 5.01. San Diego, United States).

Quantitative data were expressed as means and standard deviations or medians and interquartile ranges, according to their distribution, and qualitative data were expressed as absolute number and frequency (%). To compare groups we used, when appropriate, the ANOVA (analysis of variance) and Student's t,Wilcoxon, chi-squared, or Fisher's exact tests for two-group comparisons.

Data on cell culture were analyzed using the nonparametric Mann-Whitney test for unpaired data. Modulation of signaling pathways between groups was assessed per pathway by twoway ANOVA that also takes into account the experiment effect.

A p-value < 0.05 was considered statistically significant.

### 2. RESULTS

### Population

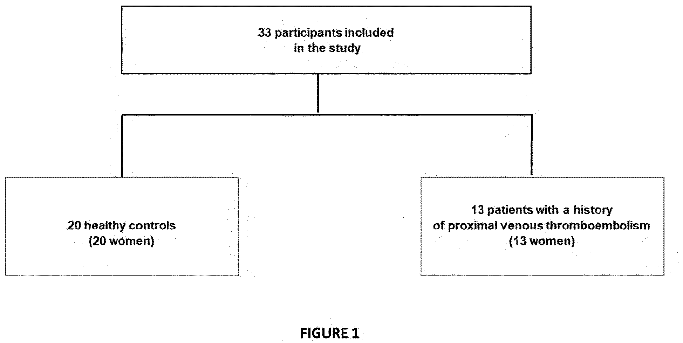

33 patients were included in the study: 20 healthy women volunteers in the group C (controls) and 13 women with a history of proximal venous thromboembolic event (VTE) in the group P (patients). The flowchart is detailed in **Figure 1** and the characteristics of the studied population are summarized in **Table 1.** For the P population, 53.8% had a history of pulmonary embolism (PE), 38% had a history of proximal deep vein thrombosis (DVT) complicated by PE and 38.5% had a history of proximal DVT; 30.8% were undergoing anticoagulant treatment and 15.4% were undergoing antiplatelet treatment. The most common anticoagulant was oral direct factor Xa inhibitor (23.1%) followed by vitamin K antagonist (7.7%). A family history of previous VTE in first-degree relatives was present in 30.8% of the patients.

**Table 1 : Characteristics of the recruited population. Quantitative variables are expressed as median with range and qualitative variables as frequency with percentage. C : controls ; P : patients ; PE : pulmonary embolism ; VTE : venous thromboembolic event; DVT : deep vein thrombosis.**

| | **Group C** | **Group P** |
|---|---|---|
| N | 20 | 13 |
| Age, years | 39.5 [22 ;49] | 36.5 [24 ;64] |
| Body Mass Index, kg/m² | 21.75 [17.5 ;29.4] | 23.55 [17.3 ;41.1] |
| Smoking habit, n, % | 4 (20%) | 4 (30,8%) |
| Previous pregnancy, n, % | 19 (95%) | 12 (85.7%) |
| Previous pregnancy complications | 0 (0%) | 1 (7.7%) |
| History of VTE events, n, % | 0 (0%) | 13 (100%) |
| History of PE, n, % | - | 7 (53.8%) |
| History of proximal DVT, n, % | - | 5 (38.5%) |
| History of proximal DVT with PE, n, % | - | 5 (38.5%) |
| History of distal DVT, n, % | | 2 (15.3%) |
| Patients undergoing antiplatelet therapy, n, % | - | 2 (15.3%) |
| Patients undergoing anticoagulant therapy, n, % | - | 4 (30.8%) |
| Vitamin K antagonists, n, % | - | 1 (7.7%) |
| Oral direct factor Xa inhibitor, n, % | - | 3 (23.1%) |
| Low molecular weight heparin, n, % | - | 0 (0%) |
| History of previous VTE in first-degree relatives, n, % | 0 (0%) | 4 (30.8%) |

**Table 2 : Biological markers at inclusion. Quantitative variables are expressed as median with range and qualitative variables as frequency with percentage. C: controls ; P : patients ; AT : antithrombin, PC : protein C ; PS : protein S.**

| | **Group C** | **Group P** |
|---|---|---|
| ***Blood cell count*** | | |
| Hemoglobin | 13.6 [10.7 ;14.9] | 13.4 [10.1 ;14.7] |
| Platelets | 273 [154 ;398] | 314 [164 ;389] |
| Leukocytes | 5.97 [3.31 ;11.77] | 6.51 [5.11 ;9.39] |

| ***Standard coagulation tests*** | | |
|---|---|---|
| aPTT, ratio | 0.98 [0.87 ;1.18] | 0.98 [0.8 ;1.17] |
| PT, ratio | 0.98 [0.9 ;1.06] | 0.99 [0.92 ;2.02] |
| Fibrinogen, g/L | 3 [2.4 ;4] | 3.1 [1.9 ;4.5] |
| D-dimers, ng/mL | 213.5 [93 ;1172] | 254.5 [89 ;636] |
| Fibrin monomers, mg/L | 5 [5 ;11] | 5 [5 ;66] |
| Factor VIII, % | 111 [85 ;206] | 186 [82 ;257] |
| Factor IX; % | 106 [86 ;132] | 119 [56 ;153] |
| Factor XI, % | 100 [72 ;142] | 109 [90 ;118] |
| Factor XII, % | 106 [79 ;112] | 103 [65 ;139] |
| AT activity, % | 98.5 [89.108] | 108 [80.122] |
| PC activity, % | 101 [76.134] | 104 [18.185] |
| PS activity, % | 73.5 [50.103] | 74 [47.109] |
| ***Conventional antiphospholipid antibodies*** | - | 0 (0%) |
| ***Genetic risk factors for thrombosis*** | | |
| *Prothrombin G20210A (rs1099963)* Heterozygous, n, % | - | 2 (15.4%) |
| *Factor V Leiden (rs6025)* Heterozygous, n, % | 2 (10%) | 1 (7.7%) |

| ***Other biological markers*** | | |
|---|---|---|
| ASAT | 19 [15.27] | 21.5 [13.48] |
| ALAT | 16.5 [10.29] | 21.5 [13.55] |
| GT | 18 [8.77] | 17 [7.246] |
| Creatinin | 69.5 [57 ;83] | 68 [50 ;82] |
| CRP | 0.6 [0.6 ;3.4] | 1.4 [0.6 ;8.7] |

### Study of P38 MAPK, AKT and ERK1/2 signaling pathways

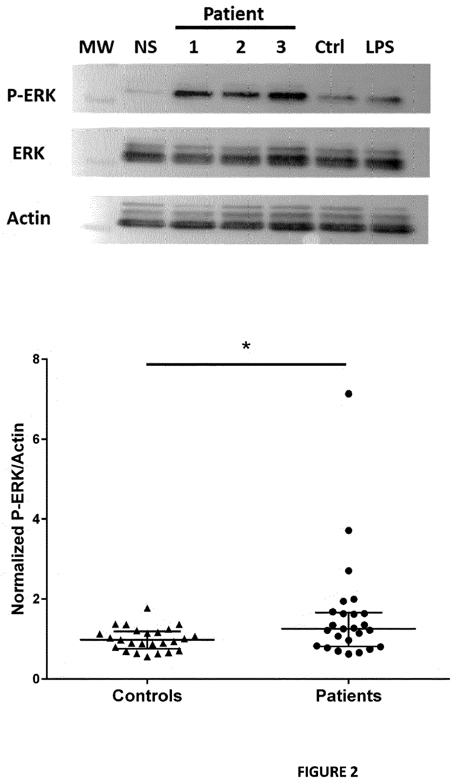

The comparison of the normalized ratio Phospho-ERK/Actin showed a significant increase of this ratio in the group patients P compared to the control group C (p=0.027) **(Figure 2A and 2B).** The same comparison for the ratio ERK/Actin did not show any significant difference corroborating an increase in the activation of ERK signaling pathway in patients with a history of proximal VTE **(Figure 2A and 2C).** All patients who had an activation of ERK pathway were analyzed again after a follow-up visit. This activation was conserved in 3 patients out of 5.

We did not show any significant difference for the two other signaling pathways: P38 MAPK and AKT.

### Receptor involved in ERK1/2 signaling pathway activation

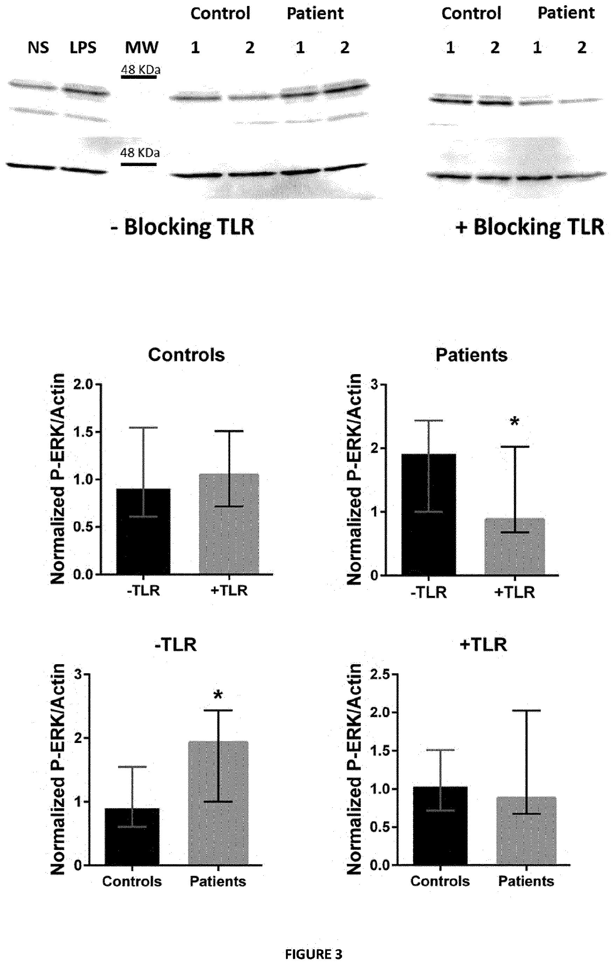

As Toll-like receptors (TLRs) play a crucial part in the pathophysiological activity of the antiphospholipid antibodies (Meroni et al. Nat Rev Rheumatol 2011;), we first studied their implication in the activation of ERK1/2 signaling pathway by purified IgG of patients with a history of proximal VTE. Interestingly, our results showed that ERK1/2 activation is impaired after blocking TLR3 **(****Figure 3****).** There is no effect after blocking TLRs 2 or 4.

These results demonstrated the crucial role of TLR3 and the ERK1/2 signaling pathway and open a new way for preventing and/or treating disorders in patients with a personal history of venous thromboembolism (VTE), in particular a history of proximal VTE, by using a TLR3 inhibitor.

### REFERENCES

Bardin N, Alessi MC, Dignat-George F, Vague IJ, Sampol J, Harlé JR, Sanmarco M. Does the anti-prothrombin antibodies measurement provide additional information in patients with thrombosis? Immunobiology. 2007;212(7):557-65. doi: 10.1016/j.imbio.2007.02.001. PMID: 17678713.
Canaud G, Bienaimé F, Tabarin F, Bataillon G, Seilhean D, Noël LH, Dragon-Durey MA, Snanoudj R, Friedlander G, Halbwachs-Mecarelli L, Legendre C, Terzi F. Inhibition of the mTORC pathway in the antiphospholipid syndrome. N Engl J Med. 2014 ;371(4):303-12. doi: 10.1056/NEJMoa1312890. PMID: 25054716.
Cuadrado MJ, Buendía P, Velasco F, Aguirre MA, Barbarroja N, Torres LA, Khamashta M, Lopez-Pedrera C. Vascular endothelial growth factor expression in monocytes from patients with primary antiphospholipid syndrome. J Thromb Haemost. 2006;4(11):2461-9. doi: 10.1111/j.1538-7836.2006.02193.x. PMID: 16968331.
Gris JC, Cochery-Nouvellon E, Bourguignon C, Mercier E, Bouvier S, Quéré I, Perez-Martin A, Molinari N, Matzner-Lober E. Reference values of coagulation assays performed for thrombophilia screening after a first venous thrombosis and their intra-patient associations. Thromb Res. 2022 Feb;210:94-103. doi: 10.1016/j.thromres.2022.01.005. PMID: 35042062
Lopez-Pedrera C, Buendía P, Cuadrado MJ, Siendones E, Aguirre MA, Barbarroja N, Montiel-Duarte C, Torres A, Khamashta M, Velasco F. Antiphospholipid antibodies from patients with the antiphospholipid syndrome induce monocyte tissue factor expression through the simultaneous activation of NF-kappaB/Rel proteins via the p38 mitogenactivated protein kinase pathway, and of the MEK-1/ERK pathway. Arthritis Rheum. 2006 ;54(1):301-11. doi: 10.1002/art.21549. PMID: 16385547.
Lopez-Pedrera C, Aguirre MA, Buendía P, Barbarroja N, Ruiz-Limon P, Collantes-Estevez E, Velasco F, Khamashta M, Cuadrado MJ. Differential expression of protease-activated receptors in monocytes from patients with primary antiphospholipid syndrome. Arthritis Rheum. 2010;62(3):869-77. doi: 10.1002/art.27299. PMID: 20131237.
Matsumoto M, Kikkawa S, Kohase M, Miyake K, Seya T. Establishment of a monoclonal antibody against human Toll-like receptor 3 that blocks double-stranded RNA-mediated signaling. Biochem Biophys Res Commun. 2002 ;293(5):1364-9. doi: 10.1016/S0006-291X(02)00380-7. PMID: 12054664.
Meroni PL, Borghi MO, Raschi E, Tedesco F. Pathogenesis of antiphospholipid syndrome: understanding the antibodies. Nat Rev Rheumatol. 2011;7(6):330-9. doi: 10.1038/nrrheum.2011.52. PMID: 21556027.
Miyakis S, Lockshin MD, Atsumi T, Branch DW, Brey RL, Cervera R, Derksen RH, DE Groot PG, Koike T, Meroni PL, Reber G, Shoenfeld Y, Tincani A, Vlachoyiannopoulos PG, Krilis SA. International consensus statement on an update of the classification criteria for definite antiphospholipid syndrome (APS). J Thromb Haemost. 2006 ;4(2):295-306. doi: 10.1111/j.1538-7836.2006.01753.x. PMID: 16420554.
Rao LV, Pendurthi UR. Regulation of tissue factor coagulant activity on cell surfaces. J Thromb Haemost. 2012 ;10(11):2242-53. doi: 10.1111/jth.12003. PMID: 23006890; PMCID: PMC3558600.Sanchez O, Benhamou Y, Bertoletti L, Constant J, Couturaud F, Delluc A, Elias A, Fischer AM, Frappe P, Gendron N, Girard P, Godier A, Gut-Gobert C, Laporte S, Mahé I, Mauge L, Meneveau N, Meyer G, Mismetti P, Parent F, Pernod G, Quéré I, Revel MP, Roy PM, Salaün PY, Smadja DM, Sevestre MA. Recommandations de bonne pratique pour la prise en charge de la maladie veineuse thromboembolique chez I'adulte. Version courte [Recommendations of good practice for the management of thromboembolic venous disease in adults. Short version]. Rev Mal Respir. 2019 ;36(2):249-283. French. doi: 10.1016/j.rmr.2019.01.003. Epub 2019 Feb 22. PMID: 30799126.
Sackett DL, Haynes RB. The architecture of diagnostic research. BMJ. 2002 2;324(7336):539-41. doi: 10.1136/bmj.324.7336.539. PMID: 11872558; PMCID: PMC1122451.
Sanmarco M, Alessi MC, Harle JR, Sapin C, Aillaud MF, Gentile S, Juhan-Vague I, Weiller PJ. Antibodies to phosphatidylethanolamine as the only antiphospholipid antibodies found in patients with unexplained thromboses. Thromb Haemost. 2001 ;85(5):800-5. PMID: 11372671.
Vega-Ostertag M, Casper K, Swerlick R, Ferrara D, Harris EN, Pierangeli SS. Involvement of p38 MAPK in the up-regulation of tissue factor on endothelial cells by antiphospholipid antibodies. Arthritis Rheum. 2005 ;52(5):1545-54. doi: 10.1002/art.21009. PMID: 15880836.

## Claims

1. A Toll-Like-Receptor 3 (TLR3) inhibitor for use in a method for preventing and/or treating disorders in patients with a personal history of venous thromboembolism (VTE), in particular a history of proximal VTE.

2. The TLR3 inhibitor for use according to claim 1, wherein the disorders are selected in the group consisting of cardiovascular disorders and/or complications that may extend to pulmonary embolism and death.

3. The TLR3 inhibitor for use according to claim 1 or claim 2, wherein the patient is an antiphospholipid antibodies (aPL) negative patient.

4. The TLR3 inhibitor for use according to anyone of claims 1 to 3, wherein the inhibitor is selected from the group consisting of a protein, a polypeptide, a peptide, a lipid, a natural or a synthetic polymer, a polysaccharide, and any compound inhibiting the ligand binding to TLR3.

5. The TLR3 inhibitor for use according to anyone of claims 1 to 4, wherein the inhibitor is selected from the group consisting of : a TLR3 blocking peptide such as DAG-P1969, a monoclonal antibody designed on the extra-cellular domain of TLR3 such as an anti-TLR3 ADCC Enhanced Antibody IPH33-1, or the antibody TLR3.7, and a molecule such as R)-2-(3-Chloro-6-fluorobenzo[b]thiophene-2-carboxamido)-3-phenylpropanoic acid or *N*-[(3-Chloro-6-fluorobenzo[*b*]thien-2-yl)carbonyl]-D-phenylalanine.

6. The TLR3 inhibitor for use according to anyone of claim 1 to 5, wherein the inhibitor suppresses downstream signaling pathways mediated by the TLR3/ligand complex, inhibiting the ERK pathways of thrombosis and haemostasis-competent cells such as monocytes activation and production of pro-inflammatory and procoagulants agents.

7. A pharmaceutical product or a composition comprising a TLR3 inhibitor and one or more active compounds selected from the group consisting of:
(i) intracellular partner inhibitor inducing the activation of the ERK signaling pathway,
(ii) anti-thrombotic agent, and
(iii) immunomodulatory agent.

8. The pharmaceutical product or composition according to claim 7, wherein the TLR 3 inhibitor is selected from the group consisting of a peptide or an antibody targeting TLR3 and the active compound is selected from the group consisting of a peptide blocking TRIF signaling by interfering with TLR-TRIF interaction, low molecular heparin (LMWH), vitamin K antagonist (VKA), direct oral anti-coagulant (DOAC), unfractionated heparin, fondaparinux, rituximab, belimumab, polyvalent gamma-globulins, and humanized immunoglobulins fragments inhibiting the binding of IgG.

9. The pharmaceutical product according to claim 7 or 8, wherein the TLR3 inhibitor and the one or more active compounds are provided for simultaneous, sequential or delayed administration.

10. The pharmaceutical product according to anyone of claim 7 to 9, for use in a patient in need thereof for preventing and/or treating disorders linked to venous thromboembolism (VTE).

11. The pharmaceutical product according to anyone of claim 6 to 8, for use according to claim 9, wherein the patient has been identified by an *in vitro* method comprising the steps of :
a) Selecting patients with a personal history of VTE, preferably with proximal VTE,
b) Screening the antiphospholipid antibodies (aPL) negative patients from individuals of step a),
c) Purifying IgG from a plasma sample of the aPL negative patient of step b),
d) Contacting the IgG of step c) with a cultured monocyte line, as THP-1 line,
e) Measuring the expression of phospho-ERK1/2, native protein ERK1/2 and actin as control, and
f) Identifying the patient that may benefit of the treatment with a TLR3 inhibitor according to the increase expression of phospho-ERK1/2 in comparison to the expression of native protein ERK1/2 and actin.

12. *An in vitro* method for identifying aPL negative patients with a personal history of VTE that may benefit of the treatment with a TLR3 inhibitor as defined in anyone of claims 1 to 6 or of the treatment with a pharmaceutical product as defined in anyone of claim 7 to 9, comprising the steps of:
a) Selecting patients with a personal history of VTE, preferably with proximal VTE,
b) Screening the antiphospholipid antibodies (aPL) negative patients from individuals of step a),
c) Purifying IgG from a plasma sample of the aPL negative patient of step b),
d) Contacting the IgG of step c) with a cultured monocyte line, as THP-1 line,
e) Measuring the expression of phospho-ERK1/2, ERK1/2 and actin as control, and
f) Identifying the patient that may benefit of the treatment with a TLR3 inhibitor according to the increase expression of phospho-ERK1/2 in comparison to the expression of ERK1/2 and actin.

13. The *in vitro* method of claim 12, wherein the step e) uses Western Blot and primary and secondary antibodies targeting phospho-ERK1/2, ERK1/2 or actin.

14. The *in vitro* method of claim 12, wherein the step e) uses a reporter gene with fluorescent or luminescent protein revealing an increase of phospho-ERK1/2 activity.

15. A kit for carrying out the *in vitro* method according to claim 13 for identifying aPL negative patients with a personal history of VTE comprising :
- A culture monocyte line, in particular THP-1 line,
- Primary and secondary antibodies targeting phospho-ERK1/2, ERK1/2 or actin,
- Reagents for cell lysis, phosphatase and protease inhibitors,
- Gel, membrane, buffers, and molecular weight ladder.

16. A kit for carrying out the *in vitro* method according to claim 14 for identifying aPL negative patients with a personal history of VTE comprising :
- A culture monocyte line, in particular THP-1 line, and
- Reporter gene with fluorescent or luminescent protein able to reveal phospho-ERK1/2 activity.
